(19) Europäisches Patentamt
European Patent Office
Office européen des brevets

(11) Veröffentlichungsnummer: **0 239 949 B1**

(12) **EUROPÄISCHE PATENTSCHRIFT**

(45) Veröffentlichungstag der Patentschrift: **10.03.93**

(51) Int. Cl.5: **C09B 61/00**, C09B 67/42, //A23L1/275

(21) Anmeldenummer: **87104571.2**

(22) Anmeldetag: **27.03.87**

(54) **Verfahren zur Herstellung von feinverteilten, pulverförmigen Carotinoidpräparaten.**

(30) Priorität: **04.04.86 DE 3611229**

(43) Veröffentlichungstag der Anmeldung:
**07.10.87 Patentblatt 87/41**

(45) Bekanntmachung des Hinweises auf die Patenterteilung:
**10.03.93 Patentblatt 93/10**

(84) Benannte Vertragsstaaten:
**BE CH DE FR GB IT LI NL**

(56) Entgegenhaltungen:
**EP-A- 0 065 193**
**DE-B- 1 211 911**
**FR-A- 2 281 961**
**US-A- 3 125 451**

**Handbook of Chemistry and Physics 62.**
**Ausgabe, Seiten C-480 und D-176**

(73) Patentinhaber: **BASF Aktiengesellschaft**
**Carl-Bosch-Strasse 38**
**W-6700 Ludwigshafen(DE)**

(72) Erfinder: **Horn, Dieter, Dr.**
**Schroederstrasse 69**
**W-6900 Heidelberg(DE)**
Erfinder: **Ouadbeck-Seeger, Hans-Juergen, Prof. Dr.**
**Heinrich-Baermann-Strasse 5**
**W-6702 Bad Duerkheim(DE)**
Erfinder: **Schaefer, Peter, Dr.**
**Im Buegen 16**
**W-6719 Kirchheim(DE)**
Erfinder: **Haehnlein, Wolfgang, Dr.**
**Im Rosengarten Ost 8**
**W-6701 Friedelsheim(DE)**

**Beschreibung**

Die Erfindung betrifft ein Verfahren zur Überführung von Carotinoiden in feinverteilte, pulverförmige Form, die insbesondere zum Färben von Lebens- und Futtermittel benötigt wird.

Die Carotinoide bilden eine Gruppe von Farbpigmenten mit gelber bis roter Farbtonnuance, die in der Natur weitverbreitet vorkommen und vielen Nahrungsmitteln eine charakteristische Färbung verleihen. Als wichtigste Vertreter dieser Stoffklasse seien β-Carotin, β-Apo-8′-carotinal, Canthaxanthin und Citranaxanthin genannt. Sowohl für die Lebensmittel-und Futtermittelindustrie als auch für die pharmazeutische Technologie stellen diese synthetisch herstellbaren Substanzen z.B. als Ersatz für künstliche Farbstoffe wichtige Farbkörper dar oder sind z.B. wegen ihrer Pro-Vitamin A Aktivität von Interesse.

Alle Carotinoide sind in Wasser unlöslich, während in Fetten und Ölen eine ebenfalls nur geringe Löslichkeit gefunden wird. Diese begrenzte Löslichkeit sowie die hohe Oxidationsempfindlichkeit behindern eine direkte Anwendung der relativ grobkörnigen bei der Synthese erhaltenen Produkte in der Einfärbung von Lebens- oder Futtermitteln, da nur eine geringe Farbausbeute erzielt werden kann und die Substanzen in grobkristalliner Form nur schlecht resorbiert werden. Diese für die praktische Verwendung der Carotinoide nachteiligen Effekte wirken sich insbesondere im wäßrigen Medium aus, da sie darin gänzlich unlöslich sind.

Zur Verbesserung der Farbausbeuten und zur Erhöhung der Resorbierbarkeit sind verschiedene Verfahren beschrieben worden, die alle das Ziel haben, die Kristallitgröße der Wirkstoffe zu verkleinern und in einen Teilchengrößenbereich von kleiner 10 μm, zu bringen. So kann z.B. nach Chimia 21, 329 (1967) β-Carotin zusammen mit Speiseöl unter Stickstoffatmosphäre in einer Kolloidmühle bis auf eine Partikelgröße von 2 bis 5 μm vermahlen werden. Gemäß Food. Technol. 12, 527 (1958) bewirkt dabei das umhüllende Öl gleichzeitig einen Oxidationsschutz für den Wirkstoff. Eine so erhaltene Suspension, die bis zu 20 oder 30 % Wirkstoff enthält, kann mit Erfolg zur Einfärbung von Fetten und Ölen benutzt werden, da trotz der geringen Löslichkeit diese ausreicht, um die Kristalle bei der üblicherweise angewandten geringen Konzentration in Lösung zu bringen.

Weit schwieriger gestaltet sich dagegen die Konfektionierung der Wirkstoffe für die Anwendung im wäßrigen Medium. Im wäßrigen Medium ist keine Löslichkeit der Carotinoide nachweisbar und die gewünschten Färbe- und Resorptionseigenschaften können nur über den möglichst feinverteilten kristallinen Zustand erzielt werden. Wünschenswert ist dabei eine Partikelgröße von kleiner 1 μm, die durch Vermahlung entweder überhaupt nicht oder nur unter Schädigung des Wirkstoffs erreichbar ist. Versuche, die Carotinoide unter Verwendung eines wasserlöslichen organischen Lösungsvermittlers, wie Alkohol oder Aceton zunächst zu lösen und dann durch Verdünnen mit Wasser feinkristallin auszufällen, scheiterten bisher an der zu geringen Löslichkeit der Carotinoide in diesen Lösungsmitteln. So beträgt z.B. die Löslichkeit von β-Carotin bei Raumtemperatur in Aceton weniger als 0,1 Gew.% und in Ethanol weniger als 0,01 Gew.%.

Andere Verfahren zur Herstellung eines Präparates mit Feinverteilung der Wirkstoffe beruhen auf deren Aufbringen auf Trägermaterialien wie Stärke, Pektin oder Trockenmilchpulver, wobei z.B. eine Lösung des Wirkstoffs in Öl gemäß DE-PS 642 307 oder Chloroform gemäß DE-PS 861 637 und CH-PS 304 023 auf die Trägermaterialien aufgesprüht wird. Die erhaltenen Präparate sind jedoch nicht universell in wäßrigen Medien dispergierbar und genügen nicht den üblichen Anforderungen an die Lagerstabilität, da die an der Oberfläche abgelagerten Wirkstoffe rasch oxidativ zerstört werden. Schließlich sind noch Verfahren zu nennen, bei denen Wirkstoffe in Form ihrer Öllösungen in Kolloide, wie Gelatine, emulsionsartig eingebettet werden, wie dies in Chimia 21, 329 (1967) und in FR-PS 1 056 114, sowie in US-PS 2 650 895 beschrieben ist. Die Wirkstoffkonzentrationen in den so hergestellten Präparaten sind wegen der geringen Öllöslichkeit der Wirkstoffe jedoch gering.

Einen gewissen Fortschritt demgegenüber stellen vorbekannte Verfahren dar, bei denen man den Wirkstoff in einem mit Wasser nicht mischbaren Lösungsmittel, vorzugsweise einem Chlorkohlenwasserstoff wie Chloroform oder Methylenchlorid löst, die Lösung durch Homogenisieren in einer Gelatine-Zucker-Lösung emulgiert und aus der Emulsion schließlich das Lösungsmittel abzieht, wobei der Wirkstoff in feinkristalliner Form freigesetzt wird. Dieses Verfahren ist in Chimia 21, 329 (1967) sowie in DE-AS 12 11 911 und DE-OS 25 34 091 beschrieben. Aus der erhaltenen Suspension wird dann durch Entwässern ein feinverteiltes Pulver gewonnen.

Dieses Verfahren hat aber den Nachteil, daß chlorierte Kohlenwasserstoffe verwendet werden müssen, um eine ausreichend hohe Wirkstoffkonzentration in der Emulsionsphase zu erzielen. Die vollständige Entfernung der chlorierten Kohlenwasserstoffe, die aus toxikologischen Gründen erforderlich ist, kann aber technisch nur schwer erzielt werden.

EP 0 239 949 B1

Diese genannten Nachteile wurden durch das in EP 0065193 beschriebene Verfahren überwunden, bei dem man zur Herstellung von feinverteilten, pulverförmigen Carotinoidpräparaten, insbesondere zum Färben von Lebens-und Futtermitteln ein Carotinoid in einem flüchtigen, mit Wasser mischbaren organischen Lösungsmittel bei Temperaturen zwischen 50°C und 240°C, gegebenenfalls unter erhöhtem Druck, innerhalb einer Zeit von weniger als 10 Sekunden löst, aus der erhaltenen molekulardispersen Lösung sofort durch schnelles Mischen mit einer wäßrigen Lösung eines quellbaren Kolloids bei Temperaturen zwischen 0 und 50°C das Carotinoid in kolloiddisperser Form ausfällt und die erhaltene Dispersion in an sich bekannter Weise von dem Lösungsmittel und dem Dispergiermedium befreit.

Ferner ist aus US 3,125,451 bekannt, Carotinoidpräparationen mit Milchprotein herzustellen, wobei das Carotinoid in einem eßbaren Lipid gelöst und dann mit z.B. Milchprotein in eine Kolloiddispersion überführt wird. Bei diesem Verfahren verbleibt das eßbare Lipid in dem fertigen Präparat und die erzielbaren Carotinoid-Konzentrationen sind sehr gering.

Im Unterschied dazu werden bei dem erfindungsgemäßen Verfahren flüchtige wassermischbare Lösungsmittel verwendet, die in der fertigen Präparation nicht mehr enthalten sind und man erhält hohe Carotinoid-Konzentrationen. Da ausdrücklich in der zitierten Literatur angegeben ist, daß Temperaturen vermieden werden müssen, bei denen z.B. die Milch koaguliert, war es überraschend, daß Milch sich für das vorliegende Verfahren als Kolloid eignet, da die z.B. ca 200°C heiße Carotinoid-Lösung mit der Milch vermischt werden kann, ohne daß eine Koagulation eintritt.

Es wurde nun überraschenderweise gefunden, daß man anstelle des quellbaren Kolloids vorteilhaft Milch oder entrahmte Milch verwenden kann.

Unter Milch wird dabei insbesondere vollfette Kuhmilch und unter entrahmter Milch teil- und vollentrahmte Milch verstanden. Die Milch kann auch mit Wasser bis zu 5fach verdünnt werden oder kann noch weitere Zusätze enthalten. Ferner kann man auch rekonstituierte Milch aus Milchpulver verwenden.

Die Möglichkeit der Verwendung von Milch war dabei aus mehreren Gründen überraschend:

1. Es ist bekannt, daß Milch durch Zugabe von Alkohol denaturiert (koaguliert) werden kann, wobei die stark pH-abhängigen Grenzkonzentrationen in der Reihenfolge Methanol, Ethanol, Propanol abfallen. Gleiches ist von Aceton bekannt (vgl. z.B. D.S. Horne, T.G. Parker, Int. J. Biol. Macromol. 3, 300 (1981).

2. Es ist weiterhin bekannt, daß Milch durch Wärmeeinwirkung zur Koagulation gebracht werden kann, wobei die Koagulationszeiten stark temperaturabhängig sind und mit steigender Temperatur abfallen (vgl. E. Dickinson, G. Stainsby, "Colloids in Food", Applied Science Publishers, London, 1982, p. 449ff).

3. Eine Kombination beider Einflußgrößen, wie sie in unserem Verfahren vorliegt, läßt daher eine erhebliche Steigerung der Denaturierungstendenz erwarten.

4. Für den Fachmann ist klar, daß ein denaturiertes, d.h. koaguliertes Milchprotein-System keine kolloide Stabilisierung eines mikrodispersen Systems bewirken kann, die eine Voraussetzung für das Gelingen des vorliegenden Verfahrens darstellt.

Es ist daher überraschend, daß selbst bei Verfahrenstemperaturen von 200°C der Feinverteilungsprozeß problemlos verläuft und ein mikrodisperses Wirkstoffmikronisat liefert, das nach Abzug des Lösungsmittels, z.B. durch Destillation, auf bekannte Weise, z.B. durch Sprühtrocknung, in eine Pulverzubereitung überführt werden kann.

Die Carotinoide, die bei der Durchführung der Erfindung eingesetzt werden können, sind die bekannten, zugänglichen, natürlichen oder synthetischen Vertreter dieser Klasse von Verbindungen, die als farbgebende Mittel brauchbar sind, z.B. Carotin, Lycopin, Bixin, Zeaxanthin, Cryptoxanthin, Citranaxanthin, Lutein, Canthaxanthin, Astaxanthin, $\beta$-Apo-4$'$-Carotinal, $\beta$-Apo-8$'$-carotinal, $\beta$-Apo-12$'$-carotinal, $\beta$-Apo-8$'$-carotinsäure sowie Ester von hydroxy- und carboxyhaltigen Vertretern dieser Gruppe, z.B. die niederen Alkylester und vorzugsweise die Methyl- und Ethylester. Besonders bevorzugt werden die bisher technisch gut zugänglichen Vertreter wie $\beta$-Carotin, Canthaxanthin, $\beta$-Apo-8$'$-carotinal und $\beta$-Apo-8$'$-Carotinsäureester.

Zur Durchführung des erfindungsgemäßen Verfahrens sind vor allem Ethanol, n-Propanol, Isopropanol, 1,2-Butandiol-1-methylether, 1,2-Propandiol-1-n-propylether oder Aceton geeignet.

Zur Erhöhung der Stabilität des Wirkstoffs gegen oxidativen Abbau ist es vorteilhaft, Stabilisatoren wie $\alpha$-Tocopherol, Lecithin, t-Butyl-hydroxytoluol, t-Butylhydroxyanisol oder Ethoxyquine zuzusetzen. Sie können entweder der Milch oder der Lösungsmittel-Phase zugesetzt werden, vorzugsweise werden sie jedoch gemeinsam mit den Farbstoffen und den tensidartigen Stabilisatoren in der Lösungsmittel-Phase gelöst. Unter Umständen kann es auch vorteilhaft sein, zusätzlich in der Lösungsmittelphase ein Öl oder einen Feststoff zu lösen, der dann gemeinsam mit den Wirkstoffen und den genannten Zusatzstoffen beim Mischen mit der wäßrigen Phase extrem feinteilig ausgefällt wird.

Man erhält eine tiefgefärbte viskose Flüssigkeit, aus der die Entfernung des Lösungsmittels je nach Siedepunkt in an sich bekannter Weise z.B. durch Destillation, gegebenenfalls unter vermindertem Druck, oder durch Extraktion mit einem mit Wasser nicht mischbaren Lösungsmittel erfolgen kann. Vorzugsweise

3

EP 0 239 949 B1

erfolgt sie jedoch gemeinsam mit der Entfernung des Wassers durch Sprühtrocknung oder Sprühgranulation.

Man erhält ein Trockenpulver, das erneut in Wasser unter Erzielung einer gleichmäßigen Feinverteilung des Wirkstoffs im Korngrößenbereich <1 μm gelöst werden kann. Im photochemischen Stabilitätstest erweist sich das so erhaltene Wirkstoff-Hydrosol trotz der Feinverteilung als außerordentlich stabil.

Alternativ kann das Wirkstoffmikronisat auch nach Einstellung eines geeigneten pH-Wertes gemeinsam mit den Milchkolloiden ausgeflockt und damit in eine Form überführt werden, aus der durch Filtrieren oder Zentrifugieren auf einfache Weise das Lösungsmittel und ein Großteil des Dispergiermediums abgetrennt werden kann. Das so gewonnene Koazervat wird dann in an sich bekannter Weise nachgetrocknet und in ein Granulat überführt.

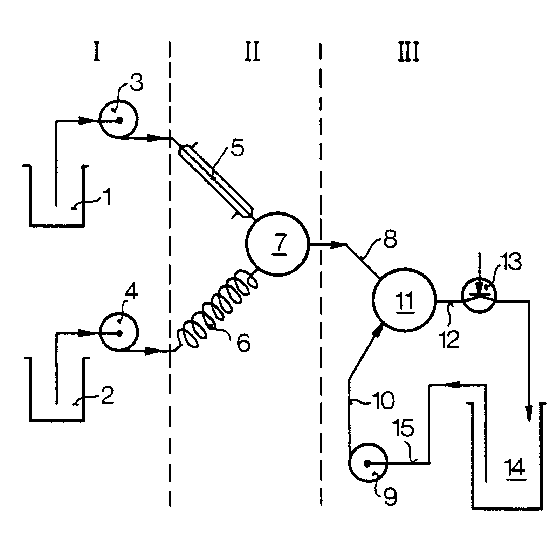

Im einzelnen führt man das erfindungsgemäße Verfahren beispielsweise mit einer Apparatur, wie sie in Fig. 1 schematisch dargestellt ist, wie folgt durch:
Die Apparatur gliedert sich in die Teile I, II und III. Teil II ist der Hochtemperaturabschnitt, während in den Teilen I und III die Temperaturen weniger als 50°C betragen.

Im Gefäß (1) wird eine Suspension des Carotinoids in dem ausgewählten Lösungsmittel, in Konzentrationen von 2 bis 20 Gew.%, bezogen auf die Mischung, gegebenenfalls unter Zusatz von 0,1 bis 10 Gew.% an Stabilisatoren, vorgelegt. Gefäß (2) enthält das Lösungsmittel ohne Beimischung des Carotinoids. Über die Pumpen (3) bzw. (4) werden die Wirkstoff-Suspensionen und das Lösungsmittel der Mischkammer (7) zugeführt, wobei das Mischungsverhältnis durch Wahl der jeweiligen Förderleistung der Pumpen vorgegeben werden kann und so gewählt wird, daß je nach Lösungsmittel und Verweilzeit eine Carotinoid-Konzentration in der Mischkammer von 0,5 bis 10 Gew.%, bezogen auf die Lösung, entsteht. Das Mischkammervolumen (7) ist so bemessen, daß bei der gewählten Förderleistung der Pumpen (3) und (4) die Verweilzeit in (7) vorzugsweise weniger als 1 Sekunde beträgt.

Das Lösungsmittel wird vor Eintritt in die Mischkammer über den Wärmetauscher (6) auf die gewünschte Temperatur gebracht, während die Wirkstoffsuspension durch Zuführung über die thermisch isolierte Zuleitung (5) bei Temperaturen unterhalb 50°C gehalten wird. Durch turbulente Mischung in (7) erfolgt im Temperaturbereich 50 bis 240°C, vorzugsweise jedoch bei 150 bis 200°C, die Lösung des Wirkstoffes und die erhaltene Lösung tritt über (8) nach kurzer Verweilzeit, vorzugsweise von weniger als einer Sekunde, in die zweite Mischkammer (11) ein, in der durch Zumischen von Milch über die Pumpe (9) und die Zuleitung (10) die Ausfällung des Wirkstoffs in kolloiddisperser Form erfolgt. Über Leitung (12) wird sodann die feinteilige Wirkstoffdispersion über das Überdruckventil (13) ausgetragen und dem Vorratsgefäß (14) zugeführt. Zur Erzielung einer möglichst hohen Wirkstoffkonzentration kann die Dispersion über die Saugleitung (15) im Kreis geführt werden.

Bei Einstellung des Überdruckventils (13) auf Drücke oberhalb ein bar können in dem neuen Verfahren sogar Lösungsmittel bei Temperaturen oberhalb ihres Siedepunktes (bei Normaldruck) verwendet werden.

Aus der Dispersion wird ein pulverförmiges Präparat in an sich bekannter Weise, z.B. gemäß den Angaben der DE-OS 25 34 091 durch Sprühtrocknen oder durch Sprühkühlen oder durch Einhüllen der Teilchen, Abtrennen und Trocknen im Wirbelbett erhalten.

Zum Sprühtrocknen wird die Dispersion entweder zuerst vom Lösungsmittel durch Destillation, vorzugsweise unter vermindertem Druck, oder durch Extraktion mit einem mit Wasser nicht mischbaren Lösungsmittel befreit oder die gesamte Mischung wird sprühgetrocknet und so Wasser und Lösungsmittel zusammen im Sprühturm abgezogen.

Am Boden des Sprühturms fällt das Carotinoidpulver bereits trocken und rieselfähig an. In manchen Fällen kann es zweckmäßig sein, die vollständige Trocknung zusätzlich in einem Wirbelbett vorzunehmen.

Anstelle der Herstellung der Pulverzubereitung durch Sprühtrocknen können auch beliebige andere Methoden angewendet werden, um die in der Wasser/Lösungsmittel-Dispersion bereits feinverteilten Carotinoide in die Pulverform zu überführen.

In den nachfolgenden Beispielen wird die Durchführung des erfindungsgemäßen Verfahrens näher erläutert.

Beispiel 1

20 g trans-$\beta$-Carotin werden in 240 g einer Lösung von 4 g Ascorbylpalmitat und 8 g dl-$\alpha$-Tocopherol in 240 g Isopropanol-Azeotrop (12 % Wasser) suspendiert und bei Einstellung des Druckbegrenzungsventils (13) auf 25 bar in der Mischkammer (7) mit 360 g Isopropanol-Azeotrop gemischt, das im Wärmetauscher (6) auf 230°C erhitzt wurde. Bei einer Dosierleistung von 2 l/h auf der Suspensionsseite und 3 l/h auf der Lösungsmittelseite beträgt die Verweilzeit in der Mischkammer (7) 0,35 Sekunden. Die dabei bei einer Temperatur von 195°C entstehende molekulardisperse Lösung wird sodann der Mischkammer (11) zugeführt, in der durch Vermischen mit 4000 g Vollmilch (Fettgehalt 3,5 %) bei einer Durchsatzgeschwindigkeit von 27 l/h das $\beta$-Carotin in kolloid-disperser Form ausgefällt wird. Es wird im Auffanggefäß (14) eine kolloid-disperse Wirkstoffdispersion mit orange-gelber Farbton-Nuance und einer Temperatur von 50°C erhalten. Die Teilchengrößenanalyse des Mikronisats durch quasi-elastische Laser-Lichtstreuung liefert eine mittlere Teilchengröße von 250 nm.

Nach Abtrennen des Lösungsmittels unter vermindertem Druck bei 50°C in einer Destillationsapparatur wird eine kolloid-disperse Wirkstoffdispersion erhalten, die durch Sprühtrocknung in ein stabiles, wasserlösliches Trockenpulver überführt werden kann.

Verfährt man wie in Beispiel 1 angegeben, verwendet jedoch die folgenden Carotinoide bzw. Milchprodukte, erhält man die in Tabelle 1 angegebenen Ergebnisse.

## Tabelle 1

| Beispiel | Milchtyp | Carotinoid | Wirkstoffgehalt des Trockenpulvers |
|---|---|---|---|
| 2 | entrahmte Milch 1,5 % Fett | $\beta$-Carotin | 3,5 % |
| 3 | vollentrahmte Milch 0,3 % Fett | " | 4,5 % |
| 4 | " jedoch mit VE-Wasser (1:2) verdünnt | " | 10 % |
| 5 | vollentrahmte Milch 0,3 % Fett) | Canthaxanthin | 4,0 % |
| 6 | " | 13-Z-Vitamin-A-Säure | 3,5 % |

Beispiel 7

Man verfährt wie in Beispiel 1 angegeben, verwendet jedoch in Mischkammer (11) nur 2000 g Vollmilch bei einer Durchsatzgeschwindigkeit von 14 l/h. Der Wirkstoffgehalt im Trockenpulver beträgt 7 %.

Beispiel 8

Man verfährt wie in Beispiel 1 angegeben, verwendet jedoch in Mischkammer (11) 4000 g einer Lösung von 150 g Trockenmilch in destilliertem Wasser.

## Patentansprüche

1. Verfahren zur Herstellung von feinverteilten, pulverförmigen Carotinoidpräparaten, in denen das Carotinoid im wesentlichen eine Teilchengröße von weniger als 0,5 Mikron besitzt, durch Lösen eines Carotinoids in einem flüchtigen, mit Wasser mischbaren, organischen Lösungsmittel bei Temperaturen zwischen 50 und 240°C, gegebenenfalls unter erhöhtem Druck, innerhalb einer Zeit von weniger als 10 Sekunden, sofortiges Ausfällen des Carotinoids in kolloiddisperser Form aus der erhaltenen molekulardispersen Lösung durch schnelles Mischen mit einer wäßrigen Dispersion eines Kolloids bei Temperaturen zwischen 0 und 50°C und Befreien der erhaltenen Dispersion in an sich bekannter Weise von dem Lösungsmittel und dem Dispergiermedium, dadurch gekennzeichnet, daß man als Kolloid Milch oder entrahmte Milch oder eine wäßrige Lösung von Trockenmilch, und als mit Wasser mischbares, organisches Lösungsmittel Aceton, 1,2-Butandiol-1-methylether, 1,2-Propandiolether, Ethanol, n-Propanol, Isopropanol oder deren Gemische verwendet.

2. Verfahren gemäß Anspruch 1, dadurch gekennzeichnet, daß die Herstellung der dispersen Carotinoidlösung und die Ausfällung des Carotinoids in feinstdisperser Form kontinuierlich in in Reihe geschalteten Mischkammern erfolgt.

**Claims**

1. A process for the preparation of a finely divided, pulverulent carotenoid preparation, in which the carotenoid essentially has a particle size of less than 0.5 micron, by dissolving a carotenoid in a volatile, water-miscible, organic solvent at from 50 to 240°C, under atmospheric or superatmospheric pressure, in less than 10 seconds, immediately precipitating the carotenoid in colloidal disperse form from the resulting molecular disperse solution by rapidly mixing with an aqueous dispersion of a colloid at from 0 to 50°C and freeing the resulting dispersion from the solvent and the dispersing medium in a conventional manner, wherein the colloid used is milk or skimmed milk or an aqueous solution of dry milk and the water-miscible organic solvent used is acetone, butane-1,2-diol 1-methyl ether, propane-1,2-diol ether, ethanol, n-propanol, isopropanol or a mixture of these.

2. A process as claimed in claim 1, wherein the preparation of the disperse carotenoid solution and the precipitation of the carotenoid in very finely disperse form are carried out continuously in mixing chambers connected in series.

**Revendications**

1. Procédé de fabrication de préparations de caroténoïde finement divisé sous forme de poudre, préparations dans lesquelles le caroténoïde se trouve essentiellement sous forme de particules de tailles inférieures à 0,5 μm, par dissolution d'un caroténoïde dans un solvant organique miscible à l'eau, volatil, à des températures comprises entre 50 et 240°C, éventuellement sous pression élevée, pendant une durée inférieure à 10 s, précipitation immédiate du caroténoïde sous forme d'une dispersion colloïdale à partir de la solution à dispersion moléculaire obtenue, par mélange rapide avec une dispersion aqueuse d'un colloïde à des températures comprises entre 0 et 50°C et séparation de la dispersion obtenue du solvant et du milieu de dispersion, de manière connue en soi, caractérisé en ce que l'on utilise, comme colloïde, du lait ou du lait écrémé ou une solution aqueuse de lait en poudre, et comme solvant organique miscible à l'eau de l'acétone, du 1,2-butanediol-1-méthyléther, du 1,2-propanedioléther, de l'éthanol, du n-propanol, de l'isopropanol ou leurs mélanges.

2. Procédé selon la revendication 1, caractérisé en ce que la fabrication de la solution dispersée de caroténoïde et la précipitation du caroténoide en forme finement dispersée est menée à bien en continu dans des caissons de mélange reliés en série.